Europäisches Patentamt

European Patent Office

Office européen des brevets

(19)

(11) Publication number: **0 172 326**

**A1**

(12)

# EUROPEAN PATENT APPLICATION

(21) Application number: **85106710.8**

(22) Date of filing: **19.02.81**

(51) Int. Cl.⁴: **A 61 L 15/03**
**A 61 K 31/715, A 61 K 33/26**
**//(A61K31/715, 31:19),**
**(A61K33/26, 31:715)**

(30) Priority: **19.02.80 US 117717**

(43) Date of publication of application:
**26.02.86 Bulletin 86/9**

(84) Designated Contracting States:
**BE CH DE FR GB IT LI NL SE**

(60) Publication number of the earlier application
in accordance with Art. 76 EPC: **0 034 504**

(71) Applicant: **Silvetti, Anthony N.**
**930 Ashland Avenue**
**River Forest Illinois 60305(US)**

(72) Inventor: **Silvetti, Anthony N.**
**930 Ashland Avenue**
**River Forest Illinois 60305(US)**

(74) Representative: **Senior, Janet et al,**
**Abel & Imray Northumberland House 303-306 High**
**Holborn**
**London WC1V 7LH(GB)**

(54) **Compositions for the treatment of wounds.**

(57) The present invention provides a composition suitable for application to skin wounds, which comprises
   (a) a starch hydrolysate having a Dextrose Equivalent less than 35, and
   (b) (i) one or more compounds selected from alphaketoglutaric acid and salts thereof, and/or
   (ii) one or more ferrous salts,
   and, if desired,
   (c) (i) one or more compounds selected from ascorbic acid and salts thereof, and/or
   (ii) one or more amino acids.

- 1 -

Combinations for the treatment of wounds.

In current medical practice, wounds and ulcers are usually irrigated and washed with either tap water, sterile distilled water or sterile normal saline solution, which is generally made isotonic with human plasma by concentration of primarily sodium chloride at a level of approximately 0.9 weight percent and with solution pH of less than about 6. It has now been learned that while such normal saline solution is an effective bacteriostatic/bactericidal agent, such solution can be a long-run detriment to the well-being of the tissue cells because they slow down or prevent healing of the wound.

Other treatment for burns and other exudative wounds and lesions has comprised using layered petralatum and gelatious protein films for sealing of the wounds to prevent serious loss of body fluid.

As described in U.S. Patent 3,812,252 which is incorporated by reference herein, a particularly effective healing treatment for wounds and skin defects such as burns, ulcers and lesions is the application of a medicinal dressing containing as an essential ingredient starch hydrolysate having Dextrose Equivalent of less than about 35. In such wound treatment the starch hydrolysate produces the formation of a film which is intimately adhered to the underlying granulation tissue and which is semipermeable to gas and fluids and provides an ideal protective cover that will reduce fluid and plasma losses and invasion by pathogenic bacteria. In addition, it appears that the starch hydrolysate provides a topical or local hyperalimentation, that is local nutrition, providing a gradual release of glucose which is particularly effective in nutrition of tissue, both damaged and nascent, which have become relatively isolated from normal blood flow nutrition. The cessation of blood flow to such an ischemic lesion can be developed in a slow and gradual form such as in the case of decubitus ulcers and statis ulcers, or may take place more acutely such as in thermo-radiation and chemical burns. In the absence of nutrition such as provided by the treatment with starch hydrolysate, the decrease in the rate of fluid delivery of nutrients brings a progressive impairment in the viability of cells and tissues which eventually leads to degeneration and death of the tissue and cells in a condition known as necrosis which is generally accompanied by bacterial, fungal, and/or viral contamination and proliferation. As further pointed out in the aforementioned patent, treatment of exudative skin wounds with a starch hydrolysate dressing produces a greatly reduced bacteria count of an infected wound and may inhibit infection of an uninfected wound. In addition, application of the starch hydrolysate to a wound or ulcer produces a film or semi-permeable membrane which allows edemic liquid to pass through while protinaceous material is retained within the body, allowing reduction in the volume of exudate in relatively clean condition. In addition, the patent teaches that nutritive agents, such as the amino acids, cystine and cysteine, and vitamins, such as ascorbic acid (vitamin C), may also be mixed or applied along with particulate starch hydrolysate to promote the formation and growth of healthy granulation tissue.

I have now found that in such starch hydrolysate treatments alphaketoglutaric acid can be used to promote development of granulation tissue by the production of collagen.

For example, compounding the starch hydrolysate composition to contain alphaketoglutaric acid, in addition to ascorbic acid or ascorbate salt, can provide a component which appears to be important for activation of the tissue enzyme, protocollagen-hydroxylase, for collagen formation.

I have also found that ferrous salts are effective in wound treatment with the starch hydrolysate: it appears that such salts also promote collagen formation.

Accordingly, the present invention provides a composition suitable for application to skin wounds, which comprises

(a)  a starch hydrolysate having a Dextrose Equivalent less than 35, and

(b)  (i)  one or more compounds selected from alphaketoglutaric acid and salts thereof, and/or

(ii) one or more ferrous salts,

and, if desired,

(c)  (i)  one or more compounds selected from ascorbic acid and salts thereof, and/or

(ii) one or more amino acids.

It should, of course, be understood that individual substances used for making the combined preparation should be suitable for application to wounds.

In this specification the term "wound" is used in a broad sense and includes, for example, burns and other damaged areas of the skin.

Topical application of alpha-keto acids and ferrous salts has already been described, respectively, in USP 4105783 and in Biddles Materia Medica and Therapeutics, page 431, 1895, but these are not concerned with the treatment of wounds and do not mention the starch hydrolysate of the composition of the present invention.

Starch hydrolysate having a Dextrose Equivalent (DE) less than about 35 suitable for application to the wound in the composition according to this invention can be prepared generally by subjecting gelatinous starch to the hydrolytic action of an acid or enzyme as more fully described in U.S. Patent 3,812,252 and U.S. Patent 3,849,194, which patents are incorporated by reference herein.

Typically, the starch hydrolysate is gamma radiated for sterilization; however, I have found that unradiated starch hydrolysate material may produce even greater healing results without radiation oxidation of the free hydroxyl groups on the polysaccharide chain which are believed to be effective in bacterial defence. Thus, advantageously, the starch hydrolysate is sterilised by a dry heat (non-radiative) sterilisation.

The composition of the invention may be present in the form of a preparation comprising the active ingredients and a carrier. It may be applied to the wound as a particulate material such as a powder, or as material such as a gel, paste, dispersion, solution or syrup.

Preferably, the starch hydrolysate used is in powder form which is commercially available from Corn Products Company under the trade name MOR-REX* typically in product grade designated Mor-Rex 1918 having a DE of approximately 10 to 13 and average molecular

* MOR-REX is a Trade Mark

0172326

weight of approximately 2000 and Mor-Rex 1908 having average molecular weight of about 4,000. Both such products are representative of starch hydrolysate materials within the preferred DE range of aproximately 5 to 25.

The alphaketoglutarate component may be added to the starch hydrolysate or the described blends· thereof at a level of approximately 1 to 3 parts by weight of alphaketoglutarate or salts thereof to 100 parts by weight starch hydrolysate.

As described in the aforementined U.S. patent 3,812,252, the wound treatment can include ascorbic acid in admixture or application along with the starch hydrolysate material to promote the formation and growth of healthy granulation tissue. Ascorbate salts such as those of sodium, potassium and calcium can also be employed, though ascorbic acid is a preferred component for blending with the starch hydrolysate powder composition at a level in the range of approximately 0.5 to 20 weight percent, preferably about 5 to 7.5 weight percent, calculated on the sum of the starch hydrolysate and ascorbic acid/ascorbate component, corresponding to a weight ratio of aproximately 20 parts starch hydrolysate to one part ascorbic acid or ascorbate salt. While ascorbic acid appears somewhat more effective than the ascorbate salts and is less readily oxidized, mixtures of ascorbic acid and ascorbate salt can also be employed in order to reduce acidity.

The use of ascorbic acid for treating burn wounds has also been described in Dutch Specification 7513892, but this specification makes no mention of the use of starch hydrolysate compositions of the present invention.

I have also found that particulate amino acid, typically in crystalline form, can advantageously be blended with the starch hydrolysate powder composition applied to the wound; suitably, the amino acid concentration in such blend can be in a range of approximately 10 to 30 parts by weight starch hydrolysate for each part by weight total amino acid, and I have found that a total amino acid concentration of approximately 5 weight percent, calculated on the sum of the starch hydrolysate and amino acid component, is effective in promoting the healing of the wound. In a particularly preferred formulation, glycine, L-proline, and L-lysine are blended with the starch hydrolysate powder composition of the present invention in order to provide a composition for application to the wound which directly combines the starch hydrolysate with the three amino acids which are believed to be most essential to the formation of collagen, the principal molecular component of both granulation tissue and connective tissue. Preferably, at least one of the foregoing group of three amino acids is blended with the starch hydrolysate composition of the invention in relative concentration of approximately 10 to 30 parts by weights starch hydrolysate component to one part total amino acid component; this composition can further comprise ascorbic acid or ascorbate salt at a level of approximately 5 parts by weight.

Table I presents the composition of a compounded starch hydrolysate blend employing particularly effective healing of pressure sores more fully described in Example 1.

TABLE I

Compounded Starch Hydrolysate Composition

| Component | Approximate Concentration in Parts by Weight/100 Parts Starch Hydrolysate |
|---|---|
| Glycine | 1 |
| L-Proline | 1 |
| L-Lysine | 1 |
| Ascorbic acid | 5 |
| Alphaketoglutaric Acid | 1 |
| $FeSO_4$ | (trace) |

Ferrous sulphate is a component in the preferred composition presented in Table I to provide $Fe^{++}$ ion which is believed to promote collagen formation; other suitable ferrous salts can also be employed.

While compounded starch hydrolysate compositions similar to that present in Table I are considered preferable, suitable compounded starch hydrolysate compositions have also been prepared by slowly adding starch hydrolysate powder to multiple amino acid solutions and then adding ascorbic acid and/or ascorbate salt as well as alphaketoglutaric acid to the solution; the solution mixture can then be slowly subjected to low temperature evaporation or to freeze-drying to obtain an alternative formulation of compounded starch hydrolysate composition in powder form for application to wounds, for example following thorough irrigation with buffered salt solution as more fully described in Example 2.

Suitable multiple amino acid solutions are, for example, those commercially available in preparations designed for intravenous use such as those available from Baxter Laboratories and Abbott Laboratories. These are aqueous solutions containing as little as 10 weight % amino acids. One such amino acid solution is supplied by Abbott Laboratories under the trade name AMINOSYN* in the form of approximately 10 weight percent total amino acid concentration with the nominal composition presented in Table II as follows:

* AMINOSYN is a Trade Mark

## TABLE II

### AMINOSYN* 10% Amino Acid Solution

Composition in mg/100 cc of solution (pH  5.3)

<u>Physiological essential amino acids</u>

| | |
|---|---|
| L-Isoleucine | 720 |
| L-Leucine | 940 |
| L-Lysine | 720 |
| L-Methionine | 400 |
| L-Phenylalanine | 440 |
| L-Threonine | 520 |
| L-Tryptophane | 160 |
| L-Valine | 800 |

<u>Physiological non essential amino acids</u>

| | |
|---|---|
| L-Tyrosine | 44 |
| L-Alanine | 1280 |
| L-Arginine | 980 |
| Glycine | 1280 |
| L-Proline | 860 |
| L-Histidine | 300 |
| L-Serine | 420 |

| | |
|---|---|
| Total amino acids | 10g/cc of solution |

To this 60 mgs of potassium metabisulphite as a preservative are added.

Suitable variations in the composition of the amino acid solution presented in Table II can be made for use in the treatment according to this invention.

In addition to selection of one or more of the amino acids presented in Table II, asparagine, aspartic acid, cystein, glutamine and glutamic acid can be added for example to the Aminosyn(TM) formulation, preferably at a level of approximately 10 to 15 mg of each of the one or more additional amino acids per 100 cc of solution. It is not essential for the amino acid compositions to include cystine or cystein as suggested for promotion of healthy granulation tissue in the aforementioned U.S. Patent 3,812,252.

In a recent paper published in the British Journal of Surgery, Vol. 63 (1976) pp. 427-430, entitled "Local Hyperalimentation of Wounds", Viljanto and Raekallio report the use of viscose cellulose sponge as a temporary cover for deep burns. In the effort to promote local cellular hyperalimentation of the wounds, the sponge was moistened by continuous slow infusion of amino acid/vitamin solutions additionally containing low levels of glucose and ascorbic acid. They report a more active granulation tissue and the disappearance of infection in the wounds so treated. Unlike the actual healing of the lesions accomplished by the starch hydrolysate composition of this invention, the temporary sponge cover for the wound was removed for skin grafting of the granulation surfaces.

The components of Vitamin B complex (dry) may, if desired, also be added to the starch hydrolysate powder mixtures.

The compounded starch hydrolysate compositions may, if desired, be cast into films and into textile dressings such as band aids and gauze.

Generally, in the preparation of wounds for treatment with starch hydrolysate dressing, the patient selected for study is carefully examined, test areas photographed, and when possible the volume of the lesion measured. Biopsy, planometric, bacterial culture, and sensitivity studies are made and thereafter the ulcer or wound is carefully surgically debrided, with all necrotic tissue removed mechanically. Where necessary, a water pulsating instrument, such as one of the instruments available under the trade name Water-Pic, has been found to facilitate the debridment of necrotic tissues and serves the purposes of saving tissues that may be viable. Enzymatic debridment can also be carried out when necessary, usually employing proteolytic enzymes such as Travase, Biozyme, collagenase, and Elase.

When the lesions are grossly infected or contaminated, treatment for a few days (one up to 3 to 4 days) with topical antibacterial agents can be employed, particularly when gram-negative organisms are present such as Pseudomonas Aeruginosa and E. Coli; suitable agents include silver nitrate (0.1% to 0.5%) solution and Silver-Sulfadiazine cream (Silvadane*, Sulfamylon*).

* Silvadane and Sulfamylon are Trade Marks.

The wound is covered with the starch hydrolysate composition preferably containing both ascorbic acid and alphaketoglutaric acid. The initial application of the starch hydrolysate material should be sufficient in amount to allow formation of a film over the entire area of the wound. The composition may, for example, be applied to a dressing or, for example, a perforated non-adhering dressing (of the TELFA* type) can be applied over the treated wound; this allows easy removal of the dressing.

The composition of the present invention may be used in a method of treating skin wounds which comprises irrigating or washing the wounds with a buffered salt solution having a pH in the range of from approximately 6 to 7.8, prior to application of the starch hydrolysate composition of the invention. This method and a combined preparation suitable for treating skin wounds by this method is more fully described in EP-A 0034504 which is incorporated by reference herein. The wound may be irrigated daily, especially with balanced salt solution buffered with phosphate to a pH of about 7.4, and then covered with a starch hydrolysate composition of the present invention; the buffered salt solution not only disinfects the wound but also promotes formation of healing granulation tissue. Further improvement of wound healing may be obtained by employing an amino acid solution to irrigate further the wound area, either before or after application of the starch hydrolysate dressing. Preferably, further irrigation of the wound is carried out, for example, by slight moisturization of the TELFA dressing with a minimal amount of amino acid solution. A roller bandage may then be applied.

The following Examples illustrate the invention but do not indicate limitation upon the scope of the claims. Throughout the Examples the starch hydrolysate used was MOR-REX (Trade Mark) as specified above.

Example 1

Preparation of starch hydrolysate composition of the invention

To each 100 gms of starch powder were added:

glycine, 1 gm;

L-proline, 1 gm.;

L-lysine 1 gm.

To this mixture was added:

5 gm of sodium ascorbate,

1 gm of alphaketoglutaric acid;

trace of ferrous sulphate.

Use of the starch hydrolysate composition in a combination treatment of burns

This is a report on the medical case of a 28 year old housewife, paraplegic for the past 5 years following traumatic injury to her spinal cord at the level of the dorsal vertebra #2. She developed a deep, heavily infected, foul smelling decubitus ulcer on the sacral area, just above the rectal region. It measured 5.5 cms x 1.5 cms by 2 cms in depth. There were 4 bacteriological species present. The lesion was treated daily by irrigation with phosphate buffered solution and then covered with the above starch hydrolysate powder of the invention.

Within 24 hours the base of the decubitus ulcer began to clean up and infection began to decrease. At 48 hours post treatment highly vascularized granulation tissue began to fill the ulcer site. Growth of granulation tissue continued and was also accompanied by centripetal growth of epithelium. By the 28th post treatment day, the ulcer site was completely healed and covered by newly formed skin without scar tissue.

Example 2

Preparation of starch hydrolysate composition

of the invention

To 100 cc of Aminosyn™ 10 % solution, 100 grams of starch hydrolysate powder was slowly and gently added, with frequent stirring; then

5 gms of ascorbic acid,

2 gms of alphaketoglutaric acid, and approximately 0.1-0.5 gm $FeSO_4$

were added to the mixture. The mixture was slowly subjected to a low temperature vacuum evaporation procedure at approximately $0^{\circ}C$ to produce a powdered mixture of all the components.

Use of the starch hydrolysate composition in a

combination treatment of burns

This is the case of a 68 year old quadriplegic woman with a deep ischiat decubitus ulcer and a deep extensive ulceration of her left heel, of 9 years duration. The areas were treated daily with phosphate buffered salt solution and then covered with the above starch hydrolysate composition.

One of the first observations made was that the applied powdered mixture did not seem to become diluted in the wound as readily as it did when the 10 % amino-acid solution was added. The mixture was slowly wetted by the wound's own fluids (serum, etc.). There has been no local nor systemic inflammatory reaction to this new type of application. It appears that the rate of healing is faster than previously noted and that the rate of wound closure (contraction of the wound) takes place much more readily and faster, which is believed to be due to the addition of the factors known to accelerate collagen formation in vitro, Fe++, $O_2$, alphaketoglutaric acid and ascorbate. By the end of 20 days both lesions were completely healed and covered by a smooth, scar-free epithelium.

CLAIMS

1.    A composition suitable for application to skin wounds, which comprises

(a)    a starch hydrolysate having a Dextrose Equivalent less than 35, and

(b)    (i)    one or more compounds selected from alphaketoglutaric acid and salts thereof, and/or

(ii) one or more ferrous salts,

and, if desired,

(c)    (i)    one or more compounds selected from ascorbic acid and salts thereof, and/or

(ii) one or more amino acids.

2.    A composition as claimed in claim 1, wherein the alphaketoglutaric acid/alphaketoglutarate component is present in an amount of from 1 to 3 parts by weight to 100 parts by weight of starch hydrolysate.

3.    A composition as claimed in claim 24 or claim 25, wherein the ferrous component comprises FeSO$_4$.

4.    A composition as claimed in any one of claims 1 to 3, containing an amino acid component in an amount of 1 part by weight per 10 to 30 parts by weight of starch hydrolysate.

5.    A composition as claimed in claim 4, wherein the amino acid component is substantially 5 % by weight of the total weight of amino acid and starch hydrolysate.

6.     A composition as claimed in any one of claims 1 to 5, containing an amino acid component comprising glycine, L-proline or L-lysine or two or all of these amino acids.

7.     A composition as claimed in claim 6, containing ascorbic acid or ascorbate salt in an amount of substantially 5 parts by weight and amino acid component in an amount of 1 part by weight per 10 to 30 parts by weight of starch hydrolysate.

8.     A composition as claimed in any one of claims 1 to 7, wherein the starch hydrolysate has a Dextrose Equivalent in the range of from 5 to 25.

9.     A composition as claimed in any one of claims 1 to 8, wherein the starch hydrolysate has been sterilised by a dry heat (non-radiative) sterilisation.

10.    A composition as claimed in any one of claims 1 to 9, which is cast into a film or present on a textile dressing.

11.    A composition as claimed in any one of claims 1 to 9, which is in the form of a powder, gel, paste, dispersion, solution or syrup.

0172326
Application number

EUROPEAN SEARCH REPORT

European Patent
Office

EP  85 10 6710

## DOCUMENTS CONSIDERED TO BE RELEVANT

| Category | Citation of document with indication, where appropriate, of relevant passages | Relevant to claim | CLASSIFICATION OF THE APPLICATION (Int. Cl 4) |
|---|---|---|---|
| D,A | US-A-3 812 252  (A.N. SILVETTI) * Column  2, lines 63-68; claims 1-5 * | 1-11 | A 61 L   15/03 A 61 K   31/715 A 61 K   33/26 // (A 61 K   31/715 A 61 K   31:19 ) (A 61 K   33/26 A 61 K   31:715) |
| A | US-A-4 105 783  (R.J. YU) * Claim 1 * | 1-11 | |
| A | FR-M-      827  (LEFRANCQ) * Abstract * | 1 | |
| A | CHEMICAL ABSTRACTS, vol. 92, no. 1, 7th January 1980, page 394, no. 4024h, Columbus, Ohio, US; W.L. McKEEHAN et al.: "Oxocarboxylic acids, pyridine nucleotide-linked oxidoreductases and serum factors in regulation of cell proliferation" & J. CELL. PHYSIOL. 1979, 101(1),9-16 * Abstract * | 1 | |
| A | THE MERCK INDEX, 9th edition, 1976, page 695, compound 5152, Merck & Co., Inc., Rahway, N.J., US; "Alpha-Ketoglutaric acid" * Page 695 * | 1 | |
| A | AMERICAN DRUG INDEX, 1975, page 228, J.B. Lippincott Co., Philadelphia, US; * Page 228 * | 3 | |

TECHNICAL FIELDS SEARCHED (Int. Cl.4)

A 61 L
A 61 K

The present search report has been drawn up for all claims

| Place of search | Date of completion of the search | Examiner |
|---|---|---|
| THE HAGUE | 29-10-1985 | PELTRE CHR. |